# DEMANDE DE BREVET EUROPEEN

(11) **EP 4 484 569 A1**
(43) Date de publication de la demande: **01.01.2025**
(21) Numéro de dépôt: 23182495.4
(22) Date de dépôt: 29.06.2023
(51) Int. Cl.: C12Q 1/04, C12Q 1/10, G01N 33/53, G01N 33/543, G01N 33/569

(54) **MILIEU RÉACTIONNEL ET MÉTHODE PERMETTANT LA DÉTECTION D'UNE E. COLI PRODUCTRICE DE SHIGATOXINE ET/OU D'UNE E. COLI ENTÉROHÉMORRAGIQUE**

(71) Demandeur: BIOMÉRIEUX, 69280 Marcy L'Etoile (FR)
(72) Inventeur: JUNILLON, Thomas, 69290 CRAPONNE (FR); MALLEN, Benoit, 69290 GREZIEUX LA VARENNE (FR)
(74) Mandataire: bioMérieux PI Groupement mandataires

(57) **Abrégé**

La présente invention concerne un milieu réactionnel gélifié pour la détection, l'identification, et/ou l'isolement d'au moins une souche d'*E*. *coli* productrice de shigatoxine comprenant
- au moins un inducteur de toxine,
- au moins un conjugué agglutinant comprenant au moins un partenaire de liaison spécifique de STX1 et/ou au moins un partenaire de liaison spécifique de STX2, couplé à une nanoparticule
- un gradient de concentration d'un composé inhibiteur des bactéries non cibles.

La présente invention concerne également la méthode associée de détection, et/ou d'isolement d'*E*. *coli* productrice de shigatoxine susceptible d'être présent dans un échantillon comprenant des entérobactéries.

## Description

### DOMAINE TECHNIQUE

La présente invention concerne le domaine du contrôle microbiologique au sens large, tel que le contrôle microbiologique d'un échantillon d'origine industrielle ou clinique. Plus particulièrement, la présente invention a trait à un milieu réactionnel et la méthode permettant la détection d'une *E. coli* productrice de shigatoxine et/ou d'une *E. coli* entérohémorragique.

### TECHNIQUE ANTERIEURE

Le contrôle microbiologique d'échantillons d'origines diverses requiert la mise en oeuvre de techniques qui permettent la détection - par exemple aux fins d'identification et/ou de dénombrement et/ou de caractérisations biochimiques - de micro-organismes et dont le rendu en termes de résultats doit être le plus rapide possible.

Dans le domaine médical, il est nécessaire de prévoir et diagnostiquer le risque infectieux : plus le diagnostic est rapide et précis, plus la prise en charge des malades est efficace et le risque de transmission minimisé. L'approche est similaire pour la santé animale dans le domaine vétérinaire.

Dans le domaine agro-alimentaire, la problématique est identique. Elle distingue cependant :
- les micro-organismes pathogènes tels que les bactéries productrices de shiga-toxines (STEC), *Salmonella, Listeria, Cronobacter, Bacillus, Staphylococcus* dont la recherche s'applique aux matières premières, produits intermédiaires, produits finis commercialisés,
- les micro-organismes non pathogènes, utilisés comme indicateurs-qualité du processus de production, des matières premières aux produits finis, tout au long de la chaîne,
- les bactéries d'intérêt technologique telles que les ferments,
- les micro-organismes marqueurs de contamination.

La détection rapide et précise des contaminations présumées (au sein des lots alimentaires) permet de les contrôler et d'engager ainsi à bref délai des actions correctives.

Techniquement, une des principales difficultés est de pouvoir isoler la bactérie recherchée pour pouvoir l'identifier. Généralement, l'analyse microbiologique se fait en deux temps. La première est une phase de détection qui peut faire appel à de nombreuses technologies telles que les milieux de culture, les immuno-essais, la biologie moléculaire. Elle peut être suivie, notamment dans le domaine agro-alimentaire, d'une phase de confirmation afin de confirmer la présence du pathogène recherché et répondre aux normes en vigueur dans ce domaine. L'étape de confirmation impose donc des étapes supplémentaires et ici encore, nécessite une étape d'isolement de la bactérie recherchée.

Ainsi, dans le cas des *E. coli* productrices de shiga-toxines, le diagnostic repose sur l'utilisation de géloses sélectives et chromogéniques pouvant comprendre du tellurite comme le CT-SMAC (Sorbitol MacConkey Agar) ou encore le CT-RMAC (Rhamnose MacConkey Agar), afin de sélectionner la pousse de certaines bactéries et de colorer les souches d'intérêt. L'inconvénient de ces méthodes est qu'ils sont très spécifiques et certaines souches d' *E. coli* productrices de shiga-toxines ne sont pas détectées. A l'inverse, certains milieux ne sont pas assez sélectifs, la présence en faible nombre d'*E*. *coli* productrices de shiga-toxines peut être cachée par les autres microorganismes très largement présents, l'isolement est alors difficile. De plus, pour évaluer la pathogénicité de ces souches, il est nécessaire de détecter la présence de facteur de virulence. En effet, leur pathogénicité implique l'expression de plusieurs gènes de virulence, notamment *stx* codant pour les deux types de shiga-toxines : STX1 et STX2. Ces deux toxines agissent en inhibant la synthèse protéique dans les cellules eucaryotes, ce qui *in fine* provoque l'apoptose. Actuellement, afin d'identifier les *E. coli* productrices de shiga-toxines avec précision, il est indispensable de réaliser une PCR pour le gène *stx.*

Il existe donc un réel besoin de mettre au point un milieu et une méthode fiable et rapide d'isolement et d'identification de bactéries cibles et notamment des STEC et des EHEC.

### RESUME DE L'INVENTION

La présente invention concerne un milieu réactionnel gélifié pour la détection, l'identification, et/ou l'isolement d'au moins une souche d'E. *coli* productrice de shigatoxine comprenant
- au moins un inducteur de toxine,
- au moins un conjugué agglutinant comprenant au moins un partenaire de liaison spécifique de STX1 et/ou au moins un partenaire de liaison spécifique de STX2, couplé à une nanoparticule
- un gradient de concentration d'un composé inhibiteur des bactéries non cibles.

De manière tout à fait avantageuse, le milieu selon l'invention permet d'isoler des STEC ou des EHEC parmi une multitude d'*E*. *coli.*

Un autre objet de l'invention concerne la préparation d'un milieu réactionnel selon l'invention comprenant les étapes suivantes :
- mise en contact d'un conjugué agglutinant avec un milieu de culture gélifiant
- déposer sur une zone du milieu de culture gélifié, au moins un composé inhibiteur des bactéries non-cibles comprenant du tellurite; ledit composé inhibiteur diffusant et formant un gradient de concentration d'inhibition autour de la zone de dépôt ; la concentration du gradient de concentration de tellurite étant compris entre 0 µg/ml et 100 µg/ml, préférentiellement entre 0 µg/ml et 50 µg/ml, encore plus préférentiellement entre 0 µg/ml et 30 µg/ml.

Un autre objet de l'invention concerne une méthode de détection, et/ou d'isolement d'*E*. *coli* productrice de shigatoxine susceptible d'être présent dans un échantillon comprenant des entérobactéries comprenant les étapes suivantes :
- disposer d'un milieu de culture gélifié sélectif permettant la croissance des *E*. *coli* comprenant un gradient de concentration d'un composé inhibiteur des bactéries non-cibles
- déposer l'échantillon sur ledit milieu de culture gélifié
- incuber ledit milieu dans des conditions permettant la croissance des *E*. *coli*
- isoler une *E*. *coli*
- confirmer que la dite *E*. *coli* est une *E*. *coli* productrice de shigatoxine

Préférentiellement, le milieu de culture est le milieu de culture selon l'invention.

Ainsi de façon avantageuse, la présence d'une d'*E*. *coli* productrice de shigatoxine est confirmée par la présence d'un halo autour de ladite E. coli productrice de shigatoxine présente dans la zone du gradient de concentration d'inhibition.

Ce mode de réalisation préféré a pour avantage de pouvoir détecter et isoler directement sur le milieu de culture ladite STEC ou EHEC. Cela permet un résultat immédiat.

Dans un autre mode de réalisation, la méthode permet la détection d'une STEC ou d'une EHEC. Selon ce mode de réalisation, l'*E*. *coli* productrice de shigatoxine détectée est une *E*. *coli* entérohémorragique.

Cette méthode est particulièrement avantageuse pour des échantillons chargés en flore annexe, où la quantité importante de colonies sur les boîtes de Pétri peut entraîner un risque de faux négatifs. Au regard du nombre important de colonies sur la boite et la faible représentation du micro-organisme cible, la personne prélevant les colonies à des fins de confirmation, peut ne jamais prélever le micro-organisme cible. La probabilité d'isoler une STEC est alors très largement augmenté en présence du gradient du composé inhibiteur. La présente invention propose donc une méthode de détection et d'isolement des STEC permettant d'éviter les faux négatifs et permet un gain de temps considérable.

### DESCRIPTION DES FIGURES

La figure 1 est une photo d'un milieu de culture témoin sans gradient de concentration de composé inhibiteur sur lequel a été ensemencé par épuisement un échantillon « reblochon ».
La figure 2 est une photo d'un milieu de culture témoin sans gradient de composé inhibiteur sur lequel a été ensemencé par épuisement un échantillon « reblochon » après une étape d'immuno-sélection.
La figure 3 est une photo d'un milieu de culture témoin sans gradient de composé inhibiteur sur lequel a été ensemencé par épuisement un échantillon « steak haché ». La flèche pointe un halo autour d'une colonie.
La figure 4 est une photo d'un milieu de culture témoin sans gradient de composé inhibiteur sur lequel a été ensemencé par épuisement un échantillon « steak haché » après une étape d'mmuno-sélection.
La figure 5 est une photo d'un milieu de culture comprenant un gradient d'un composé inhibiteur (mélange céfixime tellurite) sur lequel a été ensemencé par épuisement un échantillon « steak haché ».
La figure 6 est une photo d'un milieu de culture comprenant un gradient d'un composé inhibiteur (mélange céfixime tellurite) sur lequel a été ensemencé par épuisement un échantillon « steak haché » qui a préalablement été immuno sélectionné.
La figure 7 est une photo d'un milieu de culture comprenant un gradient d'un composé inhibiteur (mélange céfixime tellurite) sur lequel a été ensemencé par épuisement un échantillon « reblochon ». Les flèches pointent les halos autour de colonies.
La figure 8 est une photo d'un milieu de culture comprenant un gradient d'un composé inhibiteur (mélange céfixime tellurite) sur lequel a été ensemencé par épuisement un échantillon « reblochon » qui a préalablement été immuno sélectionné.

### DESCRIPTION DETAILLEE DE L'INVENTION

Certains termes et expressions utilisés dans le cadre de l'invention sont détaillés ci-après.

Un premier objet de l'invention concerne un milieu réactionnel gélifié pour la détection, l'identification, et/ou l'isolement d'au moins une souche d'E. *coli* productrice de shigatoxine comprenant
- au moins un inducteur de toxine,
- au moins un conjugué agglutinant comprenant au moins un partenaire de liaison spécifique de STX1 et/ou au moins un partenaire de liaison spécifique de STX2, couplé à une nanoparticule
- un gradient de concentration d'un composé inhibiteur des bactéries non cibles.

Par « milieu réactionnel », on entend un milieu comprenant tous les éléments nécessaires à l'expression d'un métabolisme, à la survie et/ou la croissance des micro-organismes. Ce milieu réactionnel peut soit être un milieu de culture microbiologique, soit être un milieu de révélation microbiologique. Dans ce dernier cas, la culture des micro-organismes peut être effectuée préalablement dans un autre milieu. Le milieu réactionnel peut également être mis en contact avec un milieu de culture gélosé. Il peut être placé sous ou sur le milieu de culture qui permet la croissance des micro-organismes cibles. Le milieu réactionnel peut être ajouté après l'incubation du milieu de culture. Préférentiellement, le milieu réactionnel est un milieu de culture microbiologique comprenant tous les éléments nécessaires à la croissance des micro-organismes.

Selon la présente invention, le milieu réactionnel est gélifié. Il se présente sous forme solide ou semi-solide. L'agar est l'agent gélifiant traditionnel utilisé en microbiologie pour la culture des micro-organismes, mais il est possible d'utiliser d'autres agents gélifiants comme par exemple, la gélatine, l'agarose ainsi que d'autres gélifiants naturels ou artificiels. Le conjugué est capable de former un réseau avec le composant cible dans un milieu gélifié. Ce réseau est visuellement détectable par la formation d'un halo sans qu'il ne soit nécessaire de diminuer la dureté d'un milieu réactionnel semi-solide classique. Ainsi il n'est pas nécessaire de modifier les propriétés physico-chimiques du milieu réactionnel, telles que la dureté, pour permettre d'une part la diffusion des composants ciblés et d'autre part la réaction avec les conjugués.

Un certain nombre de préparations sont disponibles dans le commerce, comme par exemple l'agar Columbia, la gélose Trypcase-soja, la gélose Mac Conkey, la gélose Mueller Hinton ou plus généralement celles décrites dans le Handbook of Microbiological Media. Ces milieux peuvent servir de base au milieu réactionnel selon l'invention. Le milieu réactionnel peut en outre comprendre d'éventuels additifs comme par exemple des acides aminés, des peptones, un ou plusieurs facteurs de croissance, des hydrates de carbone, des nucléotides, des minéraux, des vitamines, un ou plusieurs agents sélectifs, des inducteurs, des inducteurs de toxines, des tampons etc. Par « agent sélectif », on entend tout composé susceptible d'empêcher ou de ralentir la croissance d'un micro-organisme dit « non-cible », à savoir autre que le ou les micro-organisme(s) cible(s). Le terme « inducteur » se réfère à un composé capable d'induire l'expression d'un composé, telle une enzyme, une toxine, qui normalement resterait inexprimé. Ledit milieu réactionnel peut également comprendre un colorant. A titre indicatif, on peut citer comme colorant le bleu d'Evans, du rouge neutre, du sang de mouton, du sang de cheval, un opacifiant tel que l'oxyde de Titane, de la nitroaniline, du vert malachite, du vert brillant. Lorsque le milieu réactionnel selon l'invention comprend en outre un substrat enzymatique spécifique d'une activité enzymatique d'au moins un micro-organisme cible, on utilise de préférence un substrat chromogène et/ou fluorogène. Par «substrat chromogène et/ou fluorogène », on entend un substrat permettant la détection d'une activité enzymatique ou métabolique des micro-organismes cibles/recherchés grâce à un signal détectable. Le milieu réactionnel selon l'invention peut comporter en sus un indicateur de pH, sensible à la variation de pH induite par la consommation du substrat et révélant le métabolisme des micro-organismes cibles. Ledit indicateur de pH peut être un chromophore ou un fluorophore. On citera, comme exemple de chromophore, le bromocresol pourpre, le bleu de bromothymol, le rouge neutre, le bleu d'aniline, le bleu de bromocresol.

L'homme du métier peut également utiliser une boîte de Pétri divisée en segment, telle qu'une bi-boîte, ou une tri-boîte, permettant de comparer aisément plusieurs milieux, comprenant différents substrats ou différents mélanges sélectifs, sur lesquels un même échantillon biologique aura été déposé.

Selon la présente invention, le terme « micro-organisme » a la même signification que celle généralement admise en microbiologie et comprend notamment les bactéries gram positif ou gram négatif, les levures, les moisissures et plus généralement, les organismes unicellulaires, invisibles à l'oeil nu, qui peuvent être manipulés et multipliés en laboratoire.

Par « micro-organisme cible», on entend au moins un micro-organisme que l'on souhaite détecter et/ou identifier. Selon la présente invention le micro-organisme est choisi parmi les souches *E. coli* productrices de shiga-toxines. Les *E. coli* entérohémorragiques sont des souches représentant un sous-groupe des *Escherichia coli* productrices de Shiga toxine, qui ont le gène eae ou autres gènes pour l'adhérence aux cellules comme le gène aggR et provoquent un syndrome hémolytique et urémique. La possession de ces deux facteurs de virulence stx et eae en simultané rend ce pathovar très virulent pour l'Homme. Et notamment quand il s'agit des sérogroupes O26, O45, O80, O103, O111, 0121, O145 et O157.

Ainsi selon l'invention, le micro-organisme cible est une *E. coli* appartenant aux groupes des *E. coli* productrices de shiga-toxines ou des *E. coli entérohémorragiques* typiques portant les gènes stx et eae ou des E. coli entérohémorragiques atypiques portant les gènes stx et aggR par exemple. Les micro-organismes non cibles sont des bactéries poussant sur le milieu de culture en dehors de la zone du gradient de concentration d'un composé inhibiteur. En effet, l'échantillon étant polymicrobien, les STEC ou les EHEC potentiellement présents sont présents parmi des bactéries Gram négatives non spécifiques telles que des *E. coli* commensales.

Selon la présente invention, le milieu réactionnel comprend au moins un partenaire de liaison spécifique de STX1 et/ou STX2, couplé à une nanoparticule. STX1 et STX2 sont les toxines sécrétées par les souches STEC. Il existe en effet deux grands types de shiga-toxines : STX1 et STX2 qui elles-mêmes présentent de nombreux variants. A ce jour, STX1 a 4 sous types STX1a, STX1c, STX1d, STX1e, et STX2 en a 12: STX2a, STX2b STX2c, STX2d, STX2e, STX2f, STX2g, STX2h, STX2i, STX2j, STX2K, STX2l.

Le partenaire de liaison spécifique est choisi parmi les anticorps, tous types de fragments Fab, les protéines recombinantes, les phages, les protéines de phage, les oligonucléotides, les aptamères, les affimères ou tout autre ligand ou anti-ligand bien connu de l'homme du métier. Préférentiellement, le partenaire de liaison est choisi parmi les anticorps ou les protéines de phage. Préférentiellement l'anticorps est un anticorps monoclonal ou un fragment d'anticorps monoclonal. Le partenaire de liaison est spécifique d'un composant d'un micro-organisme cible. Le composant du micro-organisme cible est un composant relargué par ledit micro-organisme. Le composant peut ainsi être un élément issu de la surface de la bactérie tel qu'une protéine, un Lipopolysaccharide (LPS), un flagelle. Il peut également s'agir d'un élément interne à la bactérie tel que l'ARN, une protéine intra-cellulaire qui pourra être détecté lors de la mort d'une partie de la colonie bactérienne au cours de la croissance de celle-ci. Le partenaire de liaison peut aussi être spécifique d'un composant issu dudit micro-organisme. Ce composant peut être une molécule d'intérêt produite par le micro-organisme cible telle qu'une protéine, un antibiotique, une molécule de résistance à des agents antimicrobiens, des enzymes type protéases, des lipases ou glusidases. Selon la présente invention, le milieu comprend au moins un partenaire de liaison spécifique de la protéine STX1 et/ou au moins un partenaire de liaison spécifique de la protéine STX2. Selon un autre mode de réalisation, le milieu comprend au moins un partenaire de liaison spécifique d'un sous-type la protéine STX1 et/ou au moins un partenaire de liaison spécifique d'un sous-type de la protéine STX2 tel que XTX2a ou STX2d. Selon la présente invention le partenaire de liaison est couplé à une nanoparticule. Ce complexe final est appelé conjugué. Dans un même milieu réactionnel, il est possible d'avoir des conjugués ayant des partenaires de liaison de nature différente couplés à des nanoparticules elles même de nature différente.

Le terme « nanoparticule » désigne les particules de l'ordre de grandeur du nanomètre. Les nanoparticules peuvent être choisies parmi l'or, le fer, l'argent, le cuivre, le carbone, le latex, le silicium, l'aluminium. Préférentiellement, les nanoparticules sont des nanoparticules colloïdales ayant des propriétés optiques à savoir leur capacité à se distinguer lorsqu'un réseau se forme. De façon encore plus préférentielle, la nanoparticule est choisie parmi l'or, l'argent, le cuivre. Ainsi, lorsque les nanoparticules sont en or, elles changent de couleur, passant par exemple du rouge au gris lorsqu'elles forment un réseau. Lorsqu'elles ne sont pas agrégées, la longueur d'onde de la lumière absorbée est dans le rouge autour de 530nm. Lorsqu'elles sont agrégées, la longueur d'onde absorbée change du rouge au bleu/gris autour de 600 à 700nm. Dans un mode de réalisation particulier, il est possible d'utiliser ensemble plusieurs nanoparticules de couleurs différentes. Les réseaux ainsi formés permettent la distinction de plusieurs composants du micro-organisme cible.

Préférentiellement, les nanoparticules ont une taille comprise entre 10 et 200nm. Préférentiellement les nanoparticules ont une taille comprise entre 20 et 90nm, permettant une meilleure mobilité des conjugués dans le milieu réactionnel.

Avantageusement les nanoparticules permettent de diminuer la quantité nécessaire de partenaires de liaison pour la formation d'une agglutination. Ainsi, la concentration nécessaire en partenaires de liaison pour la réalisation d'un milieu réactionnel selon l'invention nécessite 100 à 1000 fois moins de partenaires de liaison qu'un milieu sans nanoparticule.

Préférentiellement, la quantité nécessaire en partenaires de liaison correspond à la quantité nécessaire pour recouvrir au minimum la moitié de la surface de la nanoparticule, et encore plus préférentiellement pour recouvrir entre le tiers et la moitié de la surface de la nanoparticule. Cette proportion permet au conjugué agglutinant de former un réseau dans le milieu réactionnel gélifié.

Avantageusement, les nanoparticules peuvent permettre de visualiser une agglutination autour d'une colonie bactérienne dont la taille ne permet pas encore d'être visible à l'oeil nu. La détection peut ainsi être plus précoce. Avantageusement, les nanoparticules peuvent permettre de visualiser une agglutination autour d'une colonie bactérienne dont l'aspect translucide ne permet pas la détection par un automate de lecture. La détection peut ainsi être facilité.

Le couplage de la nanoparticule au partenaire de liaison peut se faire soit par une fixation directe soit par une fixation indirecte. Par fixation directe, on entend la fixation par adsorption, soit par liaison covalente. Par fixation indirecte, on entend une fixation par l'interaction de ligands/anti-ligands comme par exemple la biotine/strepatividine ou d'autres couples bien connus de l'homme du métier (Nicholas G. Weich et al, 2017). En fonction du type de liaison choisi, l'homme du métier adaptera les conditions physico-chimiques du milieu réactionnel et notamment son pH.

Selon la présente invention, le conjugué est agglutinant c'est-à-dire qu'il provoque la formation d'un réseau d'agglutination en présence d'un composant d'un micro-organisme cible ou d'un composant issu dudit micro-organisme. Le composant étant multiépitope, plusieurs conjugués vont se fixer à ce composant et former une agglutination. Par agglutination, on entend le résultat d'une interaction entre au moins un composant d'un micro-organisme cible ou au moins un composant issu dudit micro-organisme avec des partenaires de liaison couplés à une nanoparticule. Les réactions d'agglutination comprennent des réactions immunologiques, telles des réactions antigène-anticorps ou plus généralement des interactions spécifiques entre deux molécules. Par cette interaction, composants et conjugués s'agrègent, adhèrent entre eux et forment un réseau dans le milieu réactionnel.

En pratique, plusieurs paramètres influencent la capacité du conjugué à être agglutinant dans un milieu gélifié comme principalement :
- la porosité du milieu gélifié
- la taille des nanoparticules
- la quantité de partenaires de liaison
- la quantité de nanoparticules.

Il conviendra donc d'adapter ces paramètres afin de permettre une agglutination satisfaisante permettant sa détection. Avantageusement, le réseau formé par ladite réaction spécifique est alors détecté soit visuellement, soit de façon automatique grâce à un système optique. La colonie du micro-organisme cible est ainsi repérée. De préférence, le réseau forme un halo dans le milieu réactionnel gélifié détectable soit visuellement soit grâce à un système optique. Le réseau ou le halo circonscrit ainsi la colonie qui peut alors être avantageusement différenciée et/ou identifiée au sein d'une population.

Dans un mode de réalisation particulier, le partenaire de liaison est un anticorps à une quantité permettant de recouvrir au minimum la moitié de la surface de la nanoparticule et préférentiellement au minimum le tiers de la surface de la nanoparticule.

Préférentiellement, dans ces variantes la nanoparticule est une nanoparticule d'or de taille comprise entre 20 et 90nm et à une concentration comprise entre 10¹⁰ et 10¹² nanoparticules/ml de milieu réactionnel.

Le milieu réactionnel selon l'invention comprend un inducteur de toxine. L'inducteur de toxine provoque un stress chez la bactérie qui va déclencher le cycle lytique des prophages dans la bactérie et donc stimuler la production de toxines qui seront libérées. De manière avantageuse, l'inducteur de toxine est choisi parmi les antibiotiques ou un stress physico-chimique. On citera, en tant qu'antibiotique, trimethoprime, sulfamethoxazole, norfloxacine, azithromycine, gemtamicine, polymyxine B, chloramphénicol, streptomycine, chlortetracycline, oxytetracyline, tylosine, mitomycine C, carbodox, oliquindox, rifampincine, imipenème, ciprofloxacine, cotrimoxazole, peniciline G, linlomycine. Dans un mode de réalisation préféré, le milieu selon l'invention comprend de la ciprofloxacine à une concentration comprise entre 0,005 et 0,030 mg/l. Dans un autre mode de réalisation préféré le milieu selon l'invention comprend de la mitomycine C à une concentration comprise entre 0.10 et 0.5 mg/l.

L'inducteur de toxine peut également être un stress physico-chimique réalisé, par exemple, par l'ajout de sel ou d'EDTA ou par une modification du pH ou encore par un stress UV. Le stress peut également être, par exemple, induit par l'ajout de noradrénaline.

Ledit micro-organisme cible est susceptible d'être présent dans un échantillon. Par « échantillon », on entend une petite partie ou petite quantité isolée d'une entité pour l'analyse.

L'échantillon peut être d'origine industrielle, soit, selon une liste non exhaustive, un prélèvement d'air, un prélèvement d'eau, un prélèvement effectué sur une surface, une pièce ou un produit manufacturé, un produit d'origine alimentaire. L'échantillon peut également correspondre à un prélèvement de fluide biologique (selles, urine, sang total, sérum, plasma, liquide céphalo-rachidien, sécrétion organique...), un prélèvement externe (peau, nez, gorge, ...) ou tissulaire ou des cellules isolées. Le prélèvement peut être utilisé tel quel ou, préalablement à l'analyse, subir une préparation de type enrichissement, extraction, concentration, purification, selon des méthodes connues de l'homme du métier. Le milieu réactionnel selon l'invention peut être d'autant plus intéressant lorsque l'échantillon est un échantillon polymicrobien ou chargé en flore annexe, comme par exemple les échantillons alimentaires tels que le fromage au lait cru ou les selles dans les échantillons cliniques.

Selon la présente invention, le milieu réactionnel comprend un gradient de concentration d'un composé inhibiteur des bactéries non cibles. Selon la présente invention le composé inhibiteur est choisi de telle sorte que les concentrations minimales inhibitrices (CMI) des bactéries non cibles telles que les entérobactéries commensales soient suffisamment inférieures à la CMI des STEC/EHEC.

On entend par « composé inhibiteur », tout composé inhibant la croissance bactérienne. Il peut s'agir d'un antibiotique, d'un colorant tel que le vert brillant, un sel tel que le chlorure de lithium, une endolysine de bactériophage. Préférentiellement le composé inhibiteur est du tellurite. Selon la présente invention, le composé inhibiteur favorise l'inhibition des souches non-STEC et non-EHEC.

On entend par « gradient » une concentration non homogène, croissante ou décroissante, d'un composé inhibiteur.

Selon les bactéries présentes dans l'échantillon polymicrobien, l'application d'un gradient de concentration permet de discriminer les microorganismes non-cibles qui ont une CMI inférieure aux microorganismes cibles. Ainsi les microorganismes non cibles sont les microorganismes capables de croître sur le milieu sans le gradient de concentration du composé inhibiteur. Les microorganismes cibles sont les microorganismes porteurs des gènes stx, soit les STEC.

Ainsi les bactéries non cibles telles que les entérobactéries commensales ont généralement une CMI inférieure aux STEC et EHEC.

Préférentiellement, lorsque le composé inhibiteur comprend du tellurite, le gradient de tellurite sur le milieu est compris entre 0 µg/ml et 100 µg/ml, préférentiellement entre 0 µg/ml et 50 µg/ml, encore plus préférentiellement entre 0 µg/ml et 30 µg/ml. Les différentes souches appartenant aux STEC et EHEC ont des sensibilités différentes aux composés inhibiteurs comme le tellurite. En effet les STEC et EHEC sont majoritairement plus résistantes au tellurite que les autres entérobactéries.

De plus, à l'intérieur même du groupe recherché comme les EHEC ou les STEC, les sérogroupes peuvent avoir une sensibilité différente, par exemple, une *E*. *coli* 0157 n'aura pas la même CMI au Céfixime-Tellurite qu'une *E. coli* 0103. Il y a également une sensibilité différente au sein même d'un sérogroupe. Ainsi une souche *E. coli* O111 n'aura pas la même CMI qu'une autre souche *d'E. co*li O111.

De manière tout à fait avantageuse, le milieu selon l'invention permet d'isoler des STEC ou des EHEC parmi une multitude d'*E*. *coli.*

Dans un mode de réalisation préféré, le composé inhibiteur est un mélange de céfixime et de tellurite. De préférence, la quantité de cefixime dans le mélange cefixime tellurite déposé est compris entre 0.20 µg et 0.30 µg et la quantité de tellurite est compris entre 12 et 13 µg. Préférentiellement le composé inhibiteur déposé sur le milieu a un volume compris entre 5 µl à 50 µl. Le composé inhibiteur peut être déposé par tout moyen comme par exemple une pipette.

Dans un mode de réalisation particulier le composé inhibiteur est contenu dans au moins un substrat apte à diffuser le composé inhibiteur sur le milieu de culture gélifié. Il peut s'agir par exemple d'une pastille en papier buvard renfermant le composé inhibiteur et apte à le diffuser.

Un autre objet de la présente invention concerne un kit de diagnostic permettant la
préparation d'un milieu réactionnel selon l'invention comprenant
- un conjugué agglutinant comprenant au moins un partenaire de liaison spécifique de STX1 et/ou au moins un partenaire de liaison spécifique de STX2, couplé à une nanoparticule
- un milieu gélifiant comprenant un inducteur de toxine
- un composé inhibiteur des bactéries des bactéries non-cibles

Avantageusement, le milieu réactionnel est fabriqué extemporanément à l'aide d'un kit selon l'invention. Cela permet l'utilisation unitaire d'un milieu réactionnel, et d'augmenter la stabilité de celui-ci. Préférentiellement, une fois fabriqué, le milieu est utilisé dans les 24 heures, encore plus préférentiellement dans les 12 heures.

Un autre objet de la présente invention concerne le procédé d'obtention d'un milieu réactionnel selon l'invention comprenant les étapes suivantes :
- mise en contact d'un conjugué agglutinant avec un milieu gélifiant pour former le milieu réactionnel.
   - déposer sur une zone du milieu de culture gélifié, au moins un composé inhibiteur des bactéries non-cibles; ledit composé inhibiteur diffusant et formant un gradient de concentration d'inhibition autour de la zone de dépôt; la concentration du gradient de tellurite étant compris entre 0 µg/ml et 100 µg/ml, préférentiellement entre 0 µg/ml et 50 µg/ml, encore plus préférentiellement entre 0 µg/ml et 30 µg/ml.

Ainsi le conjugué agglutinant est mis en contact avec un milieu gélifiant en surfusion pour former le milieu réactionnel selon l'invention. Le milieu gélifié comprend également un inducteur de toxine. L'ensemble est ensuite homogénéisé et coulé dans la boîte de Pétri.

Un composé inhibiteur des bactéries non-cibles est déposé sur une zone du milieu de culture gélifié. Le composé inhibiteur diffuse et forme un gradient d'inhibition autour de la zone de dépôt; Dans un autre mode de réalisation, le composé inhibiteur est déposé après le dépôt de l'échantillon.

Préférentiellement, le composé inhibiteur comprend un colorant qui permet de localiser son dépôt sur le milieu de culture et permettre ensuite un dépôt de l'échantillon au niveau du composé inhibiteur.

Un autre objet de la présente invention concerne une méthode de culture microbiologique in vitro, dans laquelle des micro-organismes susceptibles d'être présents dans un échantillon sont ensemencés dans ou sur un milieu de culture selon l'invention. Le milieu de culture ensemencé est incubé dans des conditions appropriées connues de l'homme de l'art. L'ensemencement est réalisé selon des techniques classiques de microbiologie.

Dans un mode préférentiel de l'invention, l'ensemencement se fait par un épuisement de l'échantillon polymicrobien sur le milieu de culture. L'échantillon est déposé au niveau du dépôt du composé inhibiteur de telle sorte que l'échantillon avec une charge maximale de microorganismes soit en contact avec le gradient de concentration d'inhibition. En effet, dans la première zone de l'isolement, il est rare de pouvoir distinguer les micro-organismes cibles des micro-organismes non-cibles du fait d'une très forte concentration des micro-organismes.

Les techniques d'ensemencement sont bien connues par l'homme du métier. Il peut s'agir d'un ensemencement en quadrants. La méthode des quadrants consiste à diviser une boîte de Pétri en deux, puis de diviser de nouveau par deux une moitié afin d'obtenir 3 quadrants de 50 %, 25 % et 25 %. Sur le plus grand quadrant une petite quantité d'inoculum est posée puis étalée. La boîte est ensuite tournée d'un quart afin d'étaler les bactéries sur un quadrant plus petit, puis de nouveau tournée d'un quart afin d'ensemencer le dernier petit quadrant. Dans ce mode de réalisation, le composé inhibiteur et l'échantillon sont déposé dans le même quadrant. Il existe évidement des variantes à cette technique. Il peut également s'agir d'un épuisement par stries multiples qui consiste à étaler l'inoculum vers le bas en stries serrées puis à nouveaux d'autres stries partant des bords des précédentes stries bord à bord.

Un autre objet de l'invention concerne une méthode de détection, et/ou d'isolement d'E. *coli* productrice de shigatoxine susceptible d'être présent dans un échantillon comprenant des entérobactéries comprenant les étapes suivantes :
- disposer d'un milieu de culture gélifié sélectif permettant la croissance des *E. coli* comprenant un gradient de concentration d'un composé inhibiteur des bactéries non-cibles
- déposer l'échantillon sur ledit milieu de culture
- incuber ledit milieu dans des conditions permettant la croissance des *E. coli*
- isoler une *E. coli*
- confirmer que la dite *E. coli* est une *E. coli* productrice de shigatoxine

Par « détection », on entend la détection à l'oeil nu ou à l'aide d'un appareil optique de l'existence d'une croissance des micro-organismes cibles de préférence des bactéries cibles. Lorsque le milieu de culture à partir duquel on souhaite détecter les micro-organismes cibles comprend un substrat chromogène ou fluorogène, la détection peut être effectuée à l'aide d'un appareil optique pour les substrats fluorogènes, ou à l'oeil nu ou à l'aide d'un appareil optique pour les substrats chromogènes. Lorsque le milieu de culture comprend un conjugué agglutinant, la détection peut se faire à l'oeil nu ou à l'aide d'un appareil optique en observant l'agglutination autour du micro- organisme cible.

Par « isolement » on entend le fait d'obtenir des colonies espacées les unes des autres.

Ainsi, d'une manière tout à fait surprenante, il a été constaté que la présence d'un gradient d'un composé inhibiteur permettait d'améliorer la détection et l'isolement d'une bactérie cible telle qu'une *E. coli* productrice de shigatoxine ou d'une *E. coli* entérohémorragique parmi une multitude d'*E*. *coli.*

Selon la présente invention, on dispose d'un milieu sélectif permettant la croissance des *E*. *coli* comprenant un gradient de concentration d'un composé inhibiteur des bactéries non cibles telles que les entérobactéries commensales. On citera comme milieu permettant la croissance des *E*. *coli* le milieu CHROMID^{®} coli, CHROMID^{®} EHEC, TBX ou encore Rainbow agar. Il convient alors d'ajouter un gradient de concentration d'un composé inhibiteur des bactéries non cibles.

Le composé inhibiteur est choisi de telle sorte qu'il permet de discriminer au sein même du groupe des *E. coli* productrice de shigatoxine des souches plus ou moins sensibles audit composé inhibiteur.

Le composé inhibiteur peut être déposé sur le milieu de culture par tout moyen tel qu'une pipette. Une fois le dépôt réalisé, le composé inhibiteur forme un gradient de concentration d'inhibition autour de la zone de dépôt. Il peut également s'agir d'un substrat diffusant le composé inhibiteur.

Dans un autre mode de réalisation, le composé inhibiteur est déposé après le dépôt de l'échantillon.

Selon la présente invention une étape de confirmation de la présence de l' *E*. *coli* productrice de shigatoxine est effectuée. Cette étape peut se faire selon les méthodes classiques de confirmation comme une méthode de biologie moléculaire ou une méthode immunologique. Dans ce mode de réalisation, la colonie, présente sur le milieu en contact avec le gradient du composé inhibiteur, est prélevée. Lorsqu'une *E. coli* a poussé sur le gradient, il y a une très forte probabilité pour qu'il s'agisse d'une *E. coli* productrice de shigatoxine. Un test de latex ou un test PCR conçu pour les microorganismes cibles peut être effectué. On citera par exemple le test SLIDEX^{®} E. coli ou le test PCR GENE-UP^{®} STEC top 6 ou GENE-UP^{®} E.coli 0157 :H7 ou encore GENE-UP^{®} STEC stx et eae.

Dans un mode de réalisation particulier, l'*E. coli* productrice de shigatoxine est une *E*. *coli* entérohémorragique.

Selon un mode préféré de l'invention, une étape d'incubation ou d'enrichissement de l'échantillon est effectuée avant dépôt sur le milieu de culture gélifié. Cette étape d'enrichissement requiert non seulement un milieu de culture *ad hoc* mais également une incubation de l'ensemble formé au moins par l'échantillon biologique et le milieu de culture à une température optimale pour permettre la croissance du/des micro-organisme(s) cible(s). L'incubation s'effectue, en général, à une température allant de 25 à 45°C durant un laps de temps prédéterminé (par exemple de 6h à 48h). Cette phase d'enrichissement requiert l'utilisation de milieux de culture, sélectifs ou non (en fonction du but recherché), qui ont pour but de promouvoir la croissance des micro-organismes cibles dans les échantillons, tout en limitant la croissance des flores non cibles. Les milieux de culture sont fréquemment utilisés dans des conteneurs de type sac en plastique stérile, dans lesquels ils sont mis en contact avec les échantillons alimentaires, cliniques ou environnementaux, aux fins de remise en suspension et enrichissement des micro-organismes recherchés. Tel que mentionné supra, cette phase d'enrichissement est nécessaire notamment afin de révéler la présence d'au moins un micro-organisme cible dans une quantité d'échantillon très variable et éventuellement très grande, par exemple de 25 grammes (g) à 375 g dilués dans un volume de milieu de culture compris entre 225 et 3375 millilitres (mL). A l'issue de cette étape d'enrichissement, un aliquote (généralement d'un volume compris entre 5 microlitres (µL) et 5 mL) est traditionnellement prélevé pour mettre en oeuvre l'étape de détection et/ou d'isolement des micro-organismes cibles.

Dans un mode avantageux de l'invention, l'échantillon est déposé et réparti sur le milieu par une technique d'épuisement.

Suite à l'ensemencement, le milieu réactionnel est incubé dans des conditions appropriées connues de l'homme de l'art.

Dans un mode préféré de l'invention, le milieu de culture est le milieu de culture selon l'invention comprenant au moins un conjugué agglutinant et un gradient d'un composé inhibiteur. Dans ce mode préféré de l'invention, la présence d'une d'*E*. *coli* productrice de shigatoxine est confirmée par la présence d'un halo autour de ladite STEC présente dans la zone du gradient d'inhibition. Ce mode de réalisation préféré a pour avantage de pouvoir détecter et isoler directement sur le milieu de culture ladite STEC ou EHEC. Cela permet un résultat immédiat.

Cette invention est particulièrement intéressante pour faciliter la détection des *E. coli* productrice de shigatoxine dans un échantillon polymicrobien. En effet, sans la présente invention qui permet de localiser la colonie d'intérêt, la méthode de référence ISO 16136 précise qu'il est nécessaire de tester jusqu'à 50 colonies par méthode moléculaire pour confirmer la présence d'un STEC ou d'un EHEC. Au regard du nombre important de colonies sur la boite et la faible représentation du micro-organisme cible, la personne prélevant les colonies à des fins de confirmation, peut ne jamais prélever le micro-organisme cible. La probabilité d'isoler une STEC est très largement augmenté en présence du gradient du composé inhibiteur à l'endroit du dépôt de l'échantillon. Cette méthode est particulièrement avantageuse pour des échantillons chargés en flore annexe, où la quantité importante de colonies sur les boîtes de Pétri peut entraîner un risque de faux négatifs.

### Exemples

### Exemple 1 : Préparation des échantillons, des géloses et ensemencement

| Matériel | Fournisseur | Reference |
|---|---|---|
| CT (céfixime tellurite) | BioMerieux | 424482 |
| TBX agar | BioMerieux | AEB622817 |
| EPT 225ml | BioMerieux | 42043 |
| EPT 9 ml | BioMerieux | 42111 |
| VIDAS-UP ESPT | BioMerieux | 30229 |

### Equipement :

- VIDAS Biomerieux Ref 4700023
- Densimat Biomerieux Ref 99234
- Plaque lumineuse
- Smasher biomerieux ref AESAP1064
- Etuve thermostatée 41.5°C

### Matrices utilisées :

- Steak haché congelé aux oignons : EXP 07/05/21 N° de lot 01973658
- Reblochon de Savoie AOP : EXP 06/02/23 N° de lot 125473226

### Souches utilisées :

- *E. coli* O157:H7; STX1+ eae+, CRA 9405024 / ATCC 43890

### Préparation des échantillons :

- Peser 25 g de matrice à tester dans un sac avec filtre
- Ajouter 225 ml de milieu EPT
- Malaxer 1 mn à l'aide d'un Smacher
- Incuber le sac 18 h à 41,5 °C dans une étuve thermostatée
- Suite à l'incubation aliquoter en tube de 9 ml.

Différents types de matrice sont testées : steaks hachés, reblochon.

### Préparation des géloses :

### - Préparation des nanoparticules d'or

Les nanoparticules de 40 nm sont fabriquées par la réduction du chlorure d'or par du citrate de sodium (méthode décrite par Turkevich and Frens en 1951). Ainsi, les nanoparticules d'or de 20 nm sont fabriquées à l'aide d'une solution de chlorure d'or diluée dans de l'eau distillée, additionnée de citrate trisodique puis portée à ébullition. La présence d'un pic d'absorbance à 517-519 nm permet de s'assurer de la bonne taille des particules. De ces particules de 20 nm sont synthétisées les particules de 40 nm. Pour cela les particules de 20 nm sont diluées en eau distillée puis portées à ébullition avec ajout de citrate trisodique et de chlorure d'or. Le pic d'absorbance est alors observé à 524-526 nm à froid, témoin de l'augmentation de la taille des particules.

### - Préparation du conjugué

Les anticorps utilisés sont l'anticorps 13C4 (ref hybridome ATCC CRL-1794) dirigé contre la toxine STX1 et l'anticorps 9E4H11 dirigé contre la toxine STX2 .

Ce mélange est adsorbé sur les nanoparticules à un pH de 8

### - Préparation du milieu de culture:

Le conjugué est ajouté dans la gélose TBX en surfusion à 50°C contenant l'inducteur de toxine à savoir la mitomycine C (Sigma M4287) à 250 ng/mL, afin d'obtenir une concentration en nanoparticules d'une DO 3. Le milieu est ensuite coulé dans les boites puis séché.

### Préparation de la souche :

- A J-1 repiquer la souche CRA 9405024 dans 9 ml d'EPT
- Incuber 18h à 41,5°C dans une étuve thermostatée
- Calibrer la souche à 0,4 McFarland à l'aide d'un densimat
- Effectuer des dilutions successives directement dans les tubes aliquotés de matrice

### Ensemencement des boîtes :

- Prendre le tube représentant la dilution 10⁵ UFC/ml de la souche d'intérêt
- Pour une partie du tube, utiliser le paramètre VIDAS ESPT2. Il s'agit d'un paramètre d'immuno-sélection permettant de sélectionner les souches d'intérêt à savoir *E.coli* O26, O45, O103, 111, O121, O145 et O157. L'échantillon immuno-sélectionné est ensuite ensemencé par épuisement des 30 µl sur la gélose STEC agar.
- Pour l'autre partie, réaliser directement un ensemencement par épuisement après dépôt de 10 µl sur la gélose STEC Agar.
- Incuber les boîtes 18-22 h à 37°C dans une étuve thermostatée

### Exemple 2 : Isolement de la souche ciblée (E. coli O157:H7; STX1+ eae+, ATCC : 43890) au sein d'une matrice steak haché et Reblochon sur un milieu de culture sans gradient

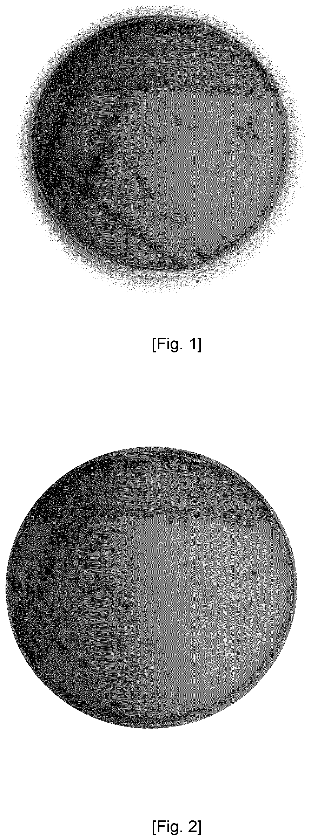

Le milieu de culture, la matrice « reblochon » et l'ensemencement sont réalisés selon l'exemple 1. Dans le cas de la matrice Reblochon qui a une flore annexe très chargée on observe figure 1 et figure 2 qu'il est difficile d'isoler les colonies caractéristiques ayant un halo. Toutes les colonies présentes dans les premiers quadrants ne sont pas identifiables car elles sont noyées au milieu de la flore annexe. Ceci est valable pour un isolement direct venant de l'enrichissement (Figure1), mais aussi après une étape d'immuno-sélection avec un VIDAS (ESP2) (Figure 2).

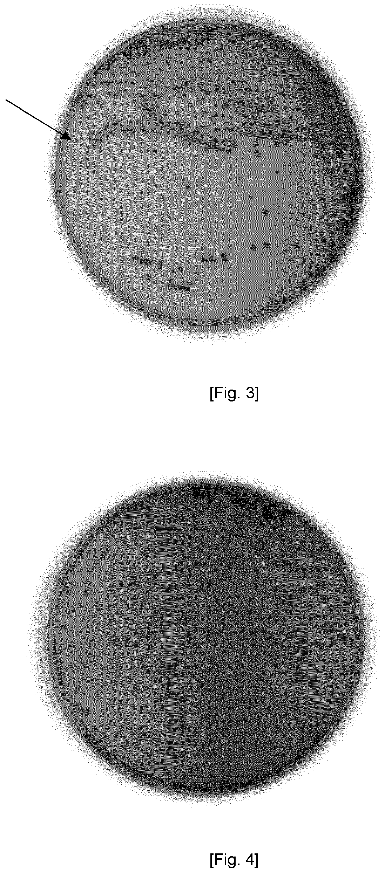

Dans le cas de la matrice « viande », présentant moins de flore annexe qu'une matrice « reblochon », on observe une colonie caractéristique par la présence d'un halo (Figure 3) après isolement direct et de multiple colonies caractéristiques avec l'étape d'immuno-sélection (Figure 4).

### Exemple 3: Isolement de la souche ciblée (E. coli O157:H7; STX1+ eae+, ATCC : 43890) au sein d'une matrice steak haché grâce à la méthode selon l'invention

### - Préparation du milieu de culture selon l'invention:

Le milieu de culture, la matrice « steak haché » sont réalisés selon l'exemple 1.

Après la dernière étape de séchage du milieu de culture on réalise les étapes suivantes :
- Représenter par un point au marqueur au dos de la boîte, la zone de dépôt prévue pour le composé inhibiteur CT. Cette zone se trouve au bord de la gélose.
- Réhydrater le flacon de supplément CT avec 400 µl d'eau déminéralisée stérile
- Déposer 10 µl de ce supplément CT sur la gélose au niveau du point précédemment définit
- Laisser sécher 10 mn afin de pouvoir ensuite utiliser la boîte pour isolement

L'ensemencement des boites est effectué selon l'exemple 1. L'échantillon de steak haché enrichi est déposé au niveau de la zone de dépôt du supplément CT.

L'incubation des boites est identique à l'exemple 1.

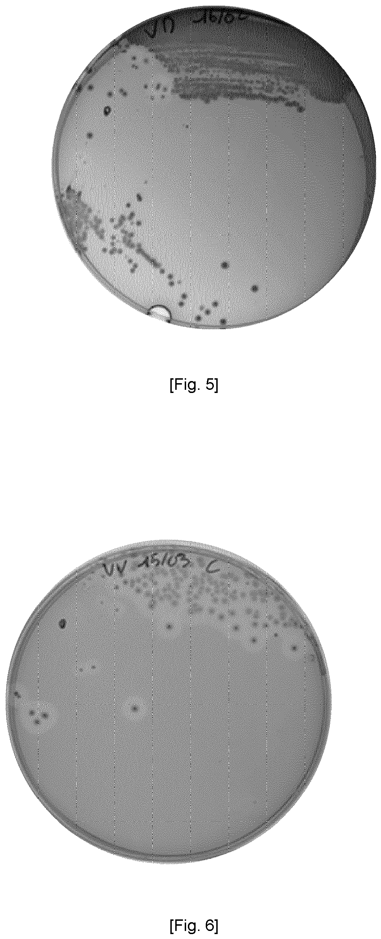

On observe sur la figure 5 un important diamètre d'inhibition autour du point de dépôt. Il apparaît ainsi des colonies isolées, et non pas en masse comme dans le cadrant suivant. Des colonies avec halo caractéristiques des souches ciblées sont visibles dans la zone du gradient de concentration d'inhibition. L'application de ce composé inhibiteur qui forme un gradient permet donc d'isoler les colonies caractéristiques.

Dans le cas d'un échantillon « steak haché » immuno sélectionné sur une boite avec un gradient, on observe figure 6 une aussi bonne détection des souches caractéristiques qu'avec la boite sans gradient avec un échantillon « steak haché » immuno-sélectionné (Figure 4).

La goutte de CT n'a pas d'effet négatif. La méthode est donc polyvalente et utilisable quel que soit la matrice.

### Exemple 4: Isolement de la souche ciblée (E. coli O157:H7; STX1+ eae+, ATCC : 43890) au sein d'une matrice reblochon grâce à la méthode selon l'invention

### Préparation du milieu de culture selon l'invention:

La préparation de gélose est réalisée selon l'exemple 1. Après la dernière étape de séchage on réalise les étapes suivantes
- Représenter par un point au marqueur au dos de la boîte la zone de dépôt prévue pour le supplément CT. Cette zone se trouve au bord de la gélose.
   - Réhydrater le flacon de supplément CT avec 400 µl d'eau déminéralisée stérile
   - Déposer 10 µl de ce supplément sur la gélose au niveau du point précédemment définit
   - Laisser sécher 10 mn afin de pouvoir ensuite utiliser la boîte pour isolement

L'ensemencement des boites est effectué selon l'exemple 1. L'échantillon de reblochon enrichie est déposé au niveau de la zone de dépôt du supplément CT.

L'incubation des boites est identique à l'exemple 1.

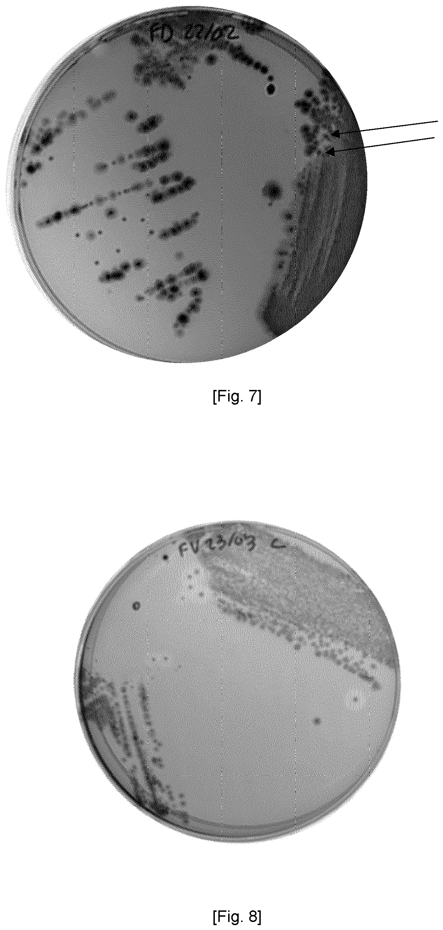

On observe sur la figure 7 un diamètre d'inhibition autour du point de dépôt. Il apparaît ainsi des colonies isolées et non pas des colonies en masse comme dans le cadrant suivant où les colonies sont beaucoup plus nombreuses. La présence du gradient de concentration permet d'obtenir des colonies isolées caractéristiques contrairement au milieu de la boite Figure 1 n'ayant pas le gradient.

On observe figure 8 pour un échantillon ayant subi l'étape d'immuno sélection, des colonies isolées caractéristiques. La présence du gradient permet d'obtenir des colonies isolées caractéristiques contrairement au milieu de la boite Figure 2 n'ayant pas le gradient.

### CONCLUSION :

L'utilisation d'un gradient de concentration d'inhibition de bactéries non-cibles permet en présence de matrice complexe et chargée en flore annexe d'inhiber lesdites bactéries non cibles afin d'isoler des colonies cibles qui sont plus résistantes.

## Revendications

1. Milieu réactionnel gélifié pour la détection, l'identification, et/ou l'isolement d'au moins une souche d'*E*. *coli* productrice de shigatoxine comprenant
- au moins un inducteur de toxine,
- au moins un conjugué agglutinant comprenant au moins un partenaire de liaison spécifique de STX1 et/ou au moins un partenaire de liaison spécifique de STX2, couplé à une nanoparticule
- un gradient de concentration d'un composé inhibiteur des bactéries non cibles

2. Milieu réactionnel gélifié selon la revendication 1 **caractérisé en ce qu'**il s'agit d'un milieu de culture microbiologique.

3. Milieu réactionnel selon l'une quelconque des revendications précédentes **caractérisé en ce que** le partenaire de liaison est choisi parmi les anticorps ou les protéines de phage.

4. Milieu selon l'une quelconque des revendications précédentes **caractérisé en ce que** le composé inhibiteur comprend du tellurite.

5. Milieu selon la revendication précédente **caractérisé en ce que** le composé inhibiteur comprend de la céfixime.

6. Milieu selon l'une quelconque des revendications précédentes **caractérisé en ce que** le gradient de concentration de tellurite sur le milieu est compris entre 0 µg/ml et 100 µg/ml, préférentiellement entre 0 µg/ml et 50 µg/ml, encore plus préférentiellement entre 0 µg/ml et 30 µg/ml.

7. Milieu selon l'une quelconque des revendications précédentes **caractérisé en ce que** la nanoparticule est une nanoparticule colloïdale ayant des propriétés optiques.

8. Milieu selon l'une quelconque des revendications précédentes **caractérisé en ce que** la nanoparticule est une nanoparticule d'or de taille entre 20 et 90 nm et est présente à une concentration comprise entre 10¹⁰ et 10¹² nanoparticules/ml de milieu réactionnel.

9. Milieu selon l'une quelconque des revendications précédentes **caractérisé en ce que** l'inducteur de toxine est un antibiotique.

10. Milieu selon l'une quelconque des revendications précédentes **caractérisé en ce que** l'inducteur de toxine est la ciprofloxacine à une concentration comprise entre 0,005 et 0,030 mg/l.

11. Milieu selon l'une quelconque des revendications précédentes **caractérisé en ce que** l'inducteur de toxine est la mitomycine C à une concentration comprise entre 0,10 mg/l à 0 , 50 mg/l.

12. Milieu selon l'une quelconque des revendications précédentes **caractérisé en ce que** le composé inhibiteur déposé a un volume compris entre de 5 µl à 50 µl.

13. Milieu selon l'une quelconque des revendications précédentes dans laquelle le composé inhibiteur est contenu dans au moins un substrat apte à diffuser ledit composé inhibiteur sur ledit milieu de culture gélifié.

14. kit de diagnostic permettant la préparation d'un milieu réactionnel selon les revendications 1 à 13 comprenant
- un conjugué agglutinant comprenant au moins un partenaire de liaison spécifique de STX1 et/ou au moins un partenaire de liaison spécifique de STX2, couplé à une nanoparticule
- un milieu gélifiant comprenant un inducteur de toxine
- un composé inhibiteur des bactéries non-cibles.

15. Préparation d'un milieu réactionnel selon les revendications 1 à 13 comprenant les étapes suivantes :
- mise en contact d'un conjugué agglutinant avec un milieu de culture gélifiant
- déposer sur une zone du milieu de culture gélifié, au moins un composé inhibiteur des bactéries non-cibles comprenant du tellurite; ledit composé inhibiteur diffusant et formant un gradient de concentration d'inhibition autour de la zone de dépôt ; la concentration du gradient de concentration de tellurite étant compris entre 0 µg/ml et 100 µg/ml, préférentiellement entre 0 µg/ml et 50 µg/ml, encore plus préférentiellement entre 0 µg/ml et 30 µg/ml

16. Préparation d'un milieu selon la revendication précédente dans laquelle le composé inhibiteur est déposé après le dépôt de l'échantillon.

17. Méthode de détection, et/ou d'isolement d'E. *coli* productrice de shigatoxine susceptible d'être présent dans un échantillon comprenant des entérobactéries comprenant les étapes suivantes :
- disposer d'un milieu de culture gélifié sélectif permettant la croissance des *E. coli* comprenant un gradient de concentration d'un composé inhibiteur des bactéries non-cibles
- déposer l'échantillon sur ledit milieu de culture gélifié
- incuber ledit milieu dans des conditions permettant la croissance des *E. coli*
- isoler une *E. coli*
- confirmer que la dite *E. coli* est une *E. coli* productrice de shigatoxine

18. Méthode selon l'une quelconque des revendications précédentes dans laquelle l'échantillon est déposé et réparti sur le milieu par une technique d'épuisement ;

19. Méthode de détection, et/ou d'isolement d'*E*. *coli* productrice de shigatoxine selon la revendication précédente **caractérisée en ce que** la méthode de confirmation est une méthode de biologie moléculaire ou une méthode immunologique.

20. Méthode de détection, et/ou d'isolement d'*E*. *coli* productrice de shigatoxine susceptible d'être présent dans un échantillon selon l'une quelconque des revendications 17 à 19 **caractérisée en ce que** le milieu de culture est le milieu de culture selon les revendications 1 à 13.

21. Méthode de détection, et/ou d'isolement d'*E*. *coli* productrice de shigatoxine susceptible d'être présent dans un échantillon selon l'une quelconque des revendications précédentes dans lesquelles l'échantillon, préalablement à son dépôt sur le milieu, est incubé dans un milieu d'enrichissement permettant la croissance d*'E*. *coli* productrice de shigatoxine.

22. Méthode selon l'une quelconque des revendications 20 ou 21 dans laquelle la présence d'une d'E. coli productrice de shigatoxine est confirmée par la présence d'un halo autour de ladite E. coli productrice de shigatoxine présente dans la zone du gradient de concentration d'inhibition

23. Méthode selon l'une quelconque des revendications 17 à 22 dans laquelle l'E. *coli* productrice de shigatoxine est une *E*. *coli* entérohémorragique.
